Europäisches Patentamt

European Patent Office

Office européen des brevets

(11) Veröffentlichungsnummer: **0 035 214**
A2

## (12) EUROPÄISCHE PATENTANMELDUNG

(21) Anmeldenummer: 81101305.1

(22) Anmeldetag: 23.02.81

(51) Int. Cl.³: **C 07 D 521/00**
// C07D277/74, C07D277/72,
C07D277/16, C07D239/56,
C07D473/38, C07D473/24

(30) Priorität: 28.02.80 CH 1590/80
05.12.80 CH 8987/80

(43) Veröffentlichungstag der Anmeldung: 09.09.81
Patentblatt 81/36

(84) Benannte Vertragsstaaten: **AT BE DE FR GB IT LU NL SE**

(71) Anmelder: **LONZA AG, Gampel/Wallis (CH)**

(72) Erfinder: **Duc, Laurent, Dr., Rue des Moulins 56, Yverdon (CH)**
Erfinder: **Boosen, Karl-Josef, Dr. Dipl.-Chem., Längereben 206, Erlach (Kanton Bern) (CH)**
Erfinder: **Marrel, Jean-Francois, Promenade des Pins 4, Yverdon (CH)**

(74) Vertreter: **Weinhold, Peter, Dr. Patentanwälte Dr. V. Schmied-Kowarzik et al, Dipl.-Ing. G. Dannenberg Dr. P. Weinhold, Dr. D. Gudel Dipl.-Ing. S. Schubert Siegfriedstr. 8, D-8000 München 40 (DE)**

(54) Verfahren zur Herstellung heterocyclischer Thioverbindungen.

(57) Das vorliegende Verfahren beschreibt die Herstellung von heterocyclischen Thioverbindungen durch Umsetzung der entsprechenden Amine mit Schwefelwasserstoff bzw. mit Thioalkoholen, mit einer Ausbeute von ~ 80% (mit wenigen Ausnahmen) und einem Reinheitsgrad von > 99%.

EP 0 035 214 A2

ACTORUM AG

- 1 -

0035214

Die vorliegende Erfindung betrifft ein Verfahren zur Herstellung heterocyclischer Thioverbindungen der allgemeinen Formel Het - S - R. Bei den erfindungsgemäss hergestellten Thioverbindungen handelt es sich also um Thioalkohole und Thioäther.

Es ist bekannt, dass heterocyclische Thioalkohole vorzugsweise durch Umsetzung von Hydroxyheterocyclen mit Phosphorpentasulfid hergestellt werden können. Auf diese Weise wird beispielsweise aus Hypoxanthin 6-Mercaptopurin erhalten. Dieses Verfahren liefert eine durchschnittliche Ausbeute an 6-Mercaptopurin von 40%.

Ziel der vorliegenden Erfindung ist ein Verfahren, welches die Erzielung hoher Ausbeuten bei gleichzeitig hohem Reinheitsgrad ermöglicht.

Erfindungsgemäss wird dies dadurch erreicht, dass man heterocyclische Aminoverbindungen in die entsprechenden Salze überführt und diese mit Schwefelwasserstoff ($H_2S$) oder mit niederen Thioalkoholen mit 1 - 6 vzw. 1 - 4 Kohlenstoffatome umsetzt. Unter dem Ausdruck Heterocyclus versteht man z.B. Reste wie Pyrimidin, Diazolin, Benzthiazol, Purin, Pyridin, Thiazol usw., sowohl substituiert als auch unsubstituiert. Ein niederer

Alkylrest beinhaltet 1 - 6 vzw. 1 - 4 Kohlenstoffatome.

Zur Salzbildung können verwendet werden Mono- oder
Dicarbonsäuren mit 1 - 6 vzw. 1 - 4 C-Atomen, wie
z.B. Ameisensäure, Essigsäure, Propionsäure, Oxalsäure,
sowie vorzugsweise aliphatische und aromatische
Sulfonsäuren, wie z.B. Benzolsulfonsäure, o-, m-,
p-Toluolsulfonsäure, Methansulfonsäure. Auch Mineralsäuren wie $H_2SO_4$, HCl sind in kleinem Maßstab verwendbar; in großen Reaktionsgefäßen tritt allerdings das
Problem der Korrosion auf.

Die zur Anwendung gelangende Menge an Verbindungen der allgemeinen Formel RSH steht in direktem stöchiometrisch-molaren Verhältnis zur Aminogruppe und wird in flüssiger Form oder als Gas dem Reaktionsmedium zugeführt. Ein Ueberschuss an RSH beeinträchtigt die Ausbeute nicht negativ.

Als Reaktionsmedium kommen Wasser oder niedere Alkohole, z.B. $C_1$-bis $C_4$-Alkohole, zum Einsatz.

Die bevorzugte Einsatzform von Schwefelwasserstoff in diesem Verfahren besteht darin, den Schwefelwasserstoff, zweckmässig in leichtem Ueberschuss, gasförmig unter Druck dem Reaktionsmedium zuzuführen. Das selbe gilt für das gasförmige $CH_3SH$; die höheren Homologen werden in flüssiger Form zugesetzt. Als bevorzugtes Reaktionsmedium wird Wasser verwendet.

Die Reaktion wird im Temperaturbereich von 80 bis 180°C, vorzugsweise bei 100 bis 160°C, durchgeführt.

Die bei diesem Verfahren erzielte Ausbeute, mit einem Reinheitsgrad von >99%, liegt in den meisten Fällen über 80%.

Gemäss dem Verfahren der vorliegenden Erfindung können heterocyclische Thioverbindungen aus den entsprechenden heterocyclischen Aminoverbindungen hergestellt werden. Solche sind zum Beispiel:

| | | |
|---|---|---|
| Thioguanin | aus | 2,6-Diaminopurin |
| 2-Mercaptothiazolin | aus | 2-Amino-Thiazolin |
| 2-Mercaptobenzothiazol | aus | 2-Amino-benzothiazol |

| | | |
|---|---|---|
| 2-Mercaptopyridin | aus | 2-Aminopyridin |
| 4-Mercaptopyridin | aus | 4-Aminopyridin |
| 2-Methylthiobenzothiazol | aus | 2-Aminobenzothiazol |
| 2-Aethylmercaptobenzo-<br>thiazol | aus | 2-Aminobenzothiazol |
| 2-Butylthiobenzthiazol | aus | 2-Aminobenzthiazol |

Beispiele

1. 6-Mercaptopurin

Zur Herstellung von 6-Mercaptopurin wurden in einem Autoklaven aus rostfreiem Stahl 50 ml $H_2O$, 4,8 g Ameisensäure
(0,104 Mol) und 7 g Adenin (0,052 Mol) vorgelegt und die
Mischung auf -80°C gekühlt. In diese so vorbereitete Vorlage wurden 6 g bei -80°C verflüssigtem $H_2S$ (0,17 Mol) zugegeben, der Autoklav verschlossen und das Reaktionsgemisch
während 18 Stunden unter Rühren mit einem Magnetrührer auf
140°C erhitzt. Nach dem Abkühlen wurde der Ueberschuss an
$H_2S$ abgeblasen und oxidativ eliminiert. Das auf 0°C abgekühlte Reaktionsprodukt wurde abgesaugt, der Filterkuchen in
70 ml $H_2O$ auspendiert und mit 12 ml konzentrierter HCl (35%)
unter Zusatz von Aktivkohle während 20 Minuten ausgekocht.
Die heisse Lösung wurde sodann filtriert, die abgefilterte
Aktivkohle mit 50 ml heissem Wasser ausgewaschen und der
pH des gesammelten Filtrats mit 20 bis 25%iger NaOH auf
4,5 eingestellt. Das nach dem Abkühlen auf 0°C anfallende 6-Mercaptopurin wurde abfiltriert, mit Wasser gewaschen und über $P_2O_5$ im Vakuum getrocknet. Die erzielte Ausbeute, mit einem Reinheitsgrad von $>99\%$, betrug 7,2 g (81%).

2. 6-Mercaptopurin

Zur Herstellung von 6-Mercaptopurin wurde wie in Beispiel 1 geschildert vorgegangen, jedoch auf eine Vorkühlung der Vorlage und auf eine Zugabe von auf $-80^{\circ}$C gekühltem $H_2S$ verzichtet. Hingegen erfolgte die Zugabe von $H_2S$ in den Autoklaven in Form von $H_2S$-Gas bei Raumtemperatur unter einem Druck von 20 bis 25 bar. Der weitere Gang des Verfahrens wurde analog Beispiel 1 durchgeführt. Die erzielte Ausbeute, mit einem Reinheitsgrad von $>99\%$, betrug 7,15 g (80,5%).

3. 2-Mercaptobenzothiazol

Man setzte wie in Beispiel 1 beschrieben 7 g (0,05 Mol) 2-Aminobenzothiazol in 50 ml $H_2O$ und 4,8 g (0,104 Mol) Ameisensäure mit 6 g (0,17 Mol) $H_2S$ während 5 Stunden bei $160^{\circ}$C um. Nach oxidativer Entfernung des $H_2S$ wurde filtriert, der Filterkuchen mit Wasser ausgewaschen und im Vakuum getrocknet. Man erhielt 8 g (96% d.Th.) 2-Mercaptobenzthiazol. Die Reinheit lag ohne Umkristallisation bei 99%.

4. Thioguanin

Zur Herstellung von Thioguanin wurden in einem Autoklaven aus rostfreiem Stahl 50 ml Wasser, 4,8 g Ameisensäure (0,104 Mol) und 7,79 g Diaminopurin (0,0518 Mol) vorgelegt und zusammen mit dem Reaktionsgefäss auf -80°C gekühlt. In diese so vorbereitete Vorlage wurden 6 g (0,17 Mol) ebenfalls auf -80°C gekühlter Schwefelwasserstoff zugegeben, der Autoklav verschlossen und die Vorlage 18 Stunden unter Rühren mit einem Magnetrührer auf 140°C erhitzt. Nach Abkühlen auf Raumtemperatur wurde der Ueberschuss an Schwefelwasserstoff durch Behandeln mit einer sauren $KMnO_4$-Lösung oxidativ entfernt und das auf 0°C abgekühlte Reaktionsprodukt abfiltriert.

Das so erhaltene Roh-Thioguanin wurde in 150 ml $NH_4OH$ (33%) unter Zusatz von Tierkohle während 10 Minuten am Rückfluss erhitzt, heiss filtriert und der Filterkuchen mit 50 ml heissem Wasser gewaschen.

Der pH des auf 20°C abgekühlten Gesamtfiltrats wurde mit konz. HCl auf pH 4,5 eingestellt, auf 0°C heruntergekühlt und bei dieser Temperatur 1 Stunde gehalten.

Der erhaltene Niederschlag wurde abfiltriert, mit 100 ml Eiswasser gewaschen, und im Vakuum (10 mbar) über $P_2O_5$ bei 80°C getrocknet.

Die erzielte Ausbeute mit einem Reinheitsgrad von 97/98% betrug 7,5 g (86%).

## 5. Thio-4-uracil

Zur Herstellung von Thio-4-uracil wurden in einem Autoklaven aus rostfreiem Stahl 50 ml Wasser, 4,8 g Ameisensäure 0,104 Mol) und 5,75 g Cytosin (0,0518 Mol) vorgelegt und zusammen mit dem Reaktionsgefäss auf -80°C gekühlt. In diese so vorbereitete Vorlage wurden 6 g (0,17 Mol) ebenfalls auf -80°C gekühlter Schwefelwasserstoff zugegeben, der Autoklav verschlossen und die vorgelegten Reaktionsteilnehmer sodann während 6 Stunden unter Rühren mit einem Magnetrührer auf 100°C erhitzt.

Nach Abkühlen auf Raumtemperatur und oxidativer Entfernung des überschüssigen Schwefelwasserstoffes wurde das Reaktionsgemisch auf 0°C gekühlt und während 2 Stunden auf dieser Temperatur gehalten. Der anschliessend abfiltrierte Rückstand wurde mit 25 ml Eiswasser gewaschen, durch Abnutschen teilweise getrocknet und sodann mit 800 ml Aethanol unter Zusatz von Aktivkohle am Rückfluss während 20 Minuten erhitzt, heiss filtriert und das Filtrat während 6 Stunden im Frigo aufbewahrt. Der hierbei entstehende Niederschlag wurde abfiltriert und im Vakuumtrockenschrank bei 50°C/10 mbar getrocknet. Das erhaltene Filtrat wurde auf 50 bis 70 ml eingeengt und über Nacht auf 0°C gehalten. Der erhaltene Niederschlag wurde abfiltriert und analog wie der erste Niederschlag getrocknet.

Die erzielte Gesamtausbeute betrug 5,7 g (86%).

6. Mercapto-2-thiazolin

Zur Herstellung von Mercapto-2-thiazolin wurden in einem
Autoklaven aus rostfreiem Stahl 50 ml Wasser, 4,8 g Ameisensäure (0,104 Mol) und 5,29 g Amino-2-thiazolin (0,0518 Mol)
vorgelegt und zusammen mit dem Reaktionsgefäss auf -80°C
gekühlt. In diese so vorbereitete Vorlage wurden 6 g (0,17
Mol) ebenfalls auf -80°C gekühlter $H_2S$ zugegeben, der Autoklav verschlossen und die Vorlage während 6 Stunden unter
Rühren auf 110°C erhitzt. Nach oxidativer Entfernung des
überschüssigen $H_2S$ wurde auf 0°C gekühlt und auf dieser Temperatur während 2 Stunden gehalten. Der abfiltrierte Rückstand wurde mit 20 ml Eiswasser gewaschen, das vereinigte
Filtrat auf ca. 20 ml eingeengt, über Nacht im Kühlschrank
aufbewahrt und filtriert.

Die vereinigten Niederschläge wurden im Vakuum über $P_2O_5$
getrocknet und ergaben eine Ausbeute von 4,7 g (76%),
Fp: 102 bis 105°C.


7. 2-(S-methylthio)benzthiazol

Zur Herstellung von 2-(S-methylthio)benzthiazol wurden in
einem Autoklaven aus rostfreiem Stahl 50 ml Wasser, 4,8 g
Ameisensäure (0,104 Mol) und 7,8 g 2-Aminobenzthiazol
(0,052 Mol) vorgelegt und zusammen mit dem Reaktionsgefäss
auf -20°C abgekühlt. In diese so vorbereitete Vorlage wurden
9,3 g $CH_3SH$ (0,193 Mol) zugegeben und das Reaktionsgemisch anschliessend während 5 Stunden unter Rühren auf 160°C erhitzt. Nach Abkühlen auf Raumtemperatur wurde der Ueberschuss an CH$_3$SH durch Behandeln mit einer sauren KMnO$_4$-Lösung oxidativ entfernt und das resultierende, aus zwei Phasen bestehende Reaktionsgemisch mit 3 x 100 ml Toluol extrahiert. Der Auszug wurde sodann über wasserfreiem, mit einigen Spatelspitzen K$_2$CO$_3$ versetztem MgSO$_4$ getrocknet, anschliessend filtriert, das Toluol abgetrieben und der Rückstand vakuumdestilliert. Kp 150 bis 160°C/10 bis 15 mbar.

Die erzielte Ausbeute, mit einem gaschromatographisch bestimmten Reinheitsgrad von 98 bis 99% und einem Smp von 45 bis 47°C, betrug 8,4 g (89%).

8. <u>2-Aethylmercapto-benzthiazol</u>

In einem Stahlautoklaven wurden 7,8 g 2-Aminobenzthiazol (0,052 Mol), 50 ml Wasser, 4,8 g Ameisensäure (0,104 Mol) und 10,6 g Aethylmercaptan (0,170 Mol) während 7 Stunden auf 140°C erhitzt. Nach dem Abkühlen wurde das Reaktionsgemisch 3 mal mit je 70 ml Toluol extrahiert und die organische Phase 3 mal mit je 50 ml 2 n Salzsäure gewaschen. Das Toluol wurde verdampft und der Rückstand destilliert.

Kp 95 bis 100°C/0,1 mbar. Ausbeute: 5,5 g (71%) 2-Aethylmercapto-benzthiazol.

Durch Vereinigung der Mutterlauge mit dem sauren Waschwasser und Einstellen des pH auf 8 erhielt man 1,8 nicht reagiertes 2-Aminobenzthiazol zurück.

0035214

9. 2-Butylmercapto-benzthiazol

Das Verfahren wurde analog Beispiel 8 durchgeführt, nur wurden anstelle von Aethylmercaptan 15,3 g n-Butylmercaptan (0,170 Mol) eingesetzt.

Kp 95 bis 100°C/0,1 mbar. Ausbeute: 2,8 g (51%) 2-Butylmercapto-benzthiazol.

Durch Aufarbeitung der Mutterlauge und des Waschwassers analog Beispiel 8 wurden 4,1 g 2-Aminobenzthiazol rückgewonnen.

Patentansprüche
_____

1. Verfahren zur Herstellung heterocyclischer Thioverbindungen der allgemeinen Formel Het - S - R, wobei R gleich H ist oder einen niederen aliphatischen Alkylrest und Het einen Heterocyclus bedeutet, aus den entsprechenden Aminoverbindungen, dadurch gekennzeichnet, daß man heterocyclische Aminoverbindungen in /überführt und diese mit einer Verbindung der Salze allgemeinen Formel HSR, worin R die oben erwähnte Bedeutung hat, bei Temperaturen von 80 bis 180°C umsetzt.

2. Verfahren nach Patentanspruch 1, dadurch gekennzeichnet, dass man als Aminsalze die Salze von aliphatischen Mono- oder Dicarbonsäuren mit 1 bis 4 C-Atomen verwendet.

3. Verfahren nach Patentanspruch 1, dadurch gekennzeichnet, dass man als Aminsalze die Salze von Sulfonsäuren verwendet.

4. Verfahren nach den Patentansprüchen 1 bis 3, dadurch gekennzeichnet, dass man bei Temperaturen von 100 bis 160°C arbeitet.